# EUROPEAN PATENT SPECIFICATION

(11) **EP 1 186 600 B1**
(45) Date of publication and mention of the grant of the patent: **10.03.2004**
(21) Application number: 01120831.1
(22) Date of filing: 30.08.2001
(51) Int. Cl.: C07D 309/04

(54) **Process for the preparation of substituted pyranes**
Verfahren zu Herstellung von substituierten Pyranen
Procédé de préparation de pyrannes sustitués

(30) Priority: 06.09.2000 EP 00119257
(43) Date of publication of application: 13.03.2002
(73) Proprietor: Roche Vitamins AG, 4070 Basel (CH)
(72) Inventor: Bonrath, Werner, 79115 Freiburg (DE); Cirillo, Fabio, 8406 Winterthur (CH)
(74) Representative: Heiroth, Ulrike Hildegard

(56) References cited:
- EP-A- 0 949 254
- BE-A- 852 918
- STEFANO SERRA: "BISABOLANE SESQUITERPENES:" SYNLETT., vol. 6, 2000, pages 890-2, XP001041818 THIEME VERLAG, STUTTGART., DE ISSN: 0936-5214

## Description

The present invention is concerned with a novel process for the preparation of substituted pyranes of the general formula I 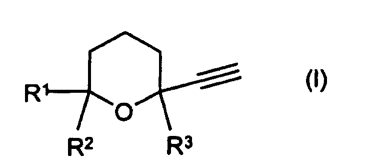 wherein R¹, R² and R³ are lower alkyl.

The compounds of formula I belong to a known class of compounds. For instance, the compound of formula I wherein each of R¹, R² and R³ is methyl, 2-ethinyl-tetrahydro-2,6,6-trimethylpyran, is a known intermediate, e.g., in the manufacture of flavours and has been prepared so far from dehydrolinalool by ring closure catalyzed by tungsten, molybdenum or polyphosphoric acid (Strickler et al., Helv. Chem. Acta 1966 49, 2055; Erman et al., Tetrahedron 1976, 34, 2981 and Belgian Patent No. 852 918). In Synlett 6, 890 - 892 (2000) 2-ethinyl-tetrahydro-2,6,6-trimethylpyran is also synthesized starting from dehydrolinalool. As catalyst boiling formic acid is used. The yield is 77%. According to EP-A 0 949 254 cyclic ethers such as tetrahydrofuran, 2-methyltetrahydrofuran, tetrahydropyran and perhydrooxepine are obtained by cyclodehydration of the corresponding alkanediols 1,4-butanediol, 1,4-pentanediol, 1,5-pentanediol and 1,6-hexanediol, respectively. The reaction is carried out in the liquid phase at a temperature from 90°C to (a temperature less than) the boiling point of the alkanediol used in the presence of crystalline aluminosilicate zeolites as the catalyst.

In accordance with the present invention it has been found that the compounds of formula I can be conveniently prepared by exposing a compound of the general formula II 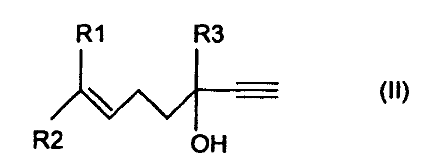 wherein R¹, R² and R³ are as above,
in the presence of a solid acid to elevated temperature or irradiation with microwaves.

The process of the present invention proceeds in high yields and in short reaction time. Further, the process of the present invention has the advantage that the use of a solvent and unwanted pressure increase in dosed reaction vessels is avoided.

The term "microwave" as used herein refers to the region of the electromagnetic spectrum having fre-quencies of 30 GHz to 300 MHz thus corresponding to wavelengths of 1 cm to 1 m. In order not to interfere with wave-lengths for Radar (1 cm - 25 cm), household or in-dustrial microwave heaters are required to operate at either 12.2 cm (2.45 GHz) or 33.3 cm (918 MHz). Thus, in a preferred embodiment of the invention, the term microwaves refers particularly to such wavelenghts. In the process of this invention, conventional microwave equipment can be used. Microwave equipment suitable in the process of this invention is supplied, e.g., by the firms MLS, Leutkirch, Germany (Lavis Multiquant 1000); or MILESTONE Inc., Monroe,CT 06468, USA (Ethos reactors). Conveniently, the irradiation in the process of this invention is carried out applying a power of irradiation of from about 600 to 1200 W, more preferably from about 800 to about 1000 W.

The solid acid used as a catalyst in the present invention is suitably a strong organic acid, such as a polymeric sulfonic acid, e.g., a polyperfluoroalkylene sulfonic acid, particularly Deloxan® ASP (Degussa, Frankfurt/M., Germany) or Nafion® NR 50 (DuPont, Wilmington, Del.,USA), or an anionic ion exchange resin such as Amberlyst® 15 (Rohm & Haas, Philadelphia, Pa.,USA); or an inorganic acid, such as sulfuric acid/silizium dioxide, or silicates such as zeolithes, e.g., Zeocat®, and Wessalith® types (Degussa), montmorillonites, e.g. Montmorillonit K 10 and KSF (Fluka, Buchs, Switzerland) and mesoporous (pore size 2-50 nm) metal-doped silica gels (Degussa)

Particularly preferred are microporous (pore size < 2 nm) zeolithes such as Wessalith®Day P and and Zeocat® types such as Zeocat Z6-05-02.

In a preferred aspect, R¹, R² and R³ are each methyl. However, any compound of the general formula I wherein R¹, R² and R³ are, independently, lower alkyl, can be prepared by the process of this invention. The term lower alkyl as used herein denotes straight or branched chain alkyl groups having up to 7 carbon atoms, such as methyl, ethyl, propyl, isopropyl, n-butyl, sek.butyl, tert.butyl, n-pentyl and isomers thereof.

If the process of the presence invention is carried out by exposing the compound of formula II to elevated temperature, the compound of formula II is heated, in the presence of a solid acid, to a temperature of about 40°C to about 100°C, preferably to about 60°C to about 100°C. However, in a preferred aspect, the present invention is concerned with the preparation of a compound of formula I wherein a compound of the general formula I is excposed to microwave irradiation.

The process according to the present invention is carried out in dry state, i.e., in the absence of solvents. Prior to exposure to elevated temperature or irradiation, the starting compound of formula II is suitably thoroughly mixed with the catalyst, e.g., by suspending the catalyst in a solution of the compound of formula II, e.g., in dichloromethane or an ether such as methyl-tert.butyl ether, with stirring and subsequent removal of the solvent or, if the catalyst is a porous material, simply adding the compound of formula II to the catalyst. The catalyst is suitably used in an amount of about 0.5 to about 10, preferably about 1 to about 2 parts of weight per part of weight of compound of formula II.

The following Examples illustrate the invention further. The reactions were carried under Ar atmosphere. The microwave equipment used was Lavis Multiquant 1000 (MLS). ETTP means 2-ethynyl-tetrahydro-2,6,6-trimethylpyran; DLL means D,L-3,7-dimethyl-6-octen-1-in-3-ol (dehydrolinalool).

### Example 1

A solution of 13.82 g (0.09 mol) of dehydrolinalool (II, R¹, R² and R³ = methyl) in 200 ml of methyl tert.butyl ether was added to 28.35 g of Zeocat® Z6-05-02 and stirred for 30 min. The reaction mixture was evaporated (40°C, 300 mbar) and the resulting solid was subjected to microwave irradiation (600 W power for 4 s). The product was separated from the solid by dissolving in 300 ml acetone and evaporating the solvent in vacuo. The crude 2-ethinyl-tetrahydro-2,6,6-trimethylpyran was obtained in 13.30 g (96.2 %) yield as yellowish liquid.

### Example 2

In analogy to the procedure of Example 1 but substituting other solid acid catalysts for Zeocat® Z6-05-02 the following results were obtained:

| catalyst | reaction time [sec] | yield ETTP [%] | amount DLL [%] | conversion [%] |
|---|---|---|---|---|
| a) Deloxan® ASP | 10 | 84.0 | 14.9 | 85.1 |
| b) Amberlys® 15 | 10 | 80.0 | 19.5 | 80.5 |
| c) Fulmont® XMP3 | 36 | 63.1 | 36.1 | 63.9 |
| d) Montmorillonit K10 | 15 | 76.4 | 20.9 | 79.1 |
| e) Montmorillonit KSF | 20 | 62.3 | 37.1 | 62.9 |
| f) Nafion® NR 50 | 63 | 32.6 | 67.4 | 32.6 |
| g) H2SO4/SiO2 | 4 | 96.6 | 3.4 | 96.6 |
| h) Wessalith® Day P | 4 | 99.1 | 0.0 | 100 |
| i) mesoporeous silica gel | 60 | 8.0 | 85.0 | 15.0 |
| j) mesoporeous silica gel | | | | |
| H+ - form | 0 | 43.0 | 55.0 | 45.0 |

### Example 3

The procedure of Examples 1 and 2 was repeated re-using the catalysts recovered from the first run in two further runs. The results were as shown below:

| catalyst | run 1 | run 2 | run 3 | run 1 | run 2 | run 2 |
|---|---|---|---|---|---|---|
| Deloxan® ASP | 84.1 | 83.9 | 82.9 | 85.1 | 84.2 | 94.3 |
| Wessalith® DayP | 99.1 | 97.8 | 97.5 | 99.1 | 97.9 | 96.3 |
| Zeocat® Z6-05-02 | 96.2 | 98.3 | 96.9 | 96.4 | 98.5 | 99.0 |

### Example 4

D,L-3,7-dimethyl-6-octen-1-in-3-ol (DDL) (0.92 g, 6 mmol) was adsorbed on the solid acid (2 g). After evaporation of the solvent (experiments using mesoporeous silica gel were carried out with a solution of DDL in methyl tert.butyl ether (MTBE); experiments using Wessalith® Day P were carried out without solvent), the solid was stirred for 60 min at room temperature, 40 °C, 60 °C, 80 °C, and 100 °C. After cooling to room temperature, the organic material was desorbed with 250 ml MTBE, and the organic layer was concentrated in vacuum (40°C, 50 mbar). The crude product was analyzed by GC. The results are summarized below:

| Catalyst | reaction temperature [°C] | yield ETTP [%] |
|---|---|---|
| Wessalith | room temperature | 8 |
| Wessalith | 40 | 29 |
| Wessalith | 60 | 77 |
| Wessalith | 80 | 71 |
| Wessalith | 100 | 69 |
| mesoporeous silica gel-H+ | room temperature | 10 |
| mesoporeous silica gel-H+ | 40 | 26 |
| mesoporeous silica gel-H+ | 60 | 89 |
| mesoporeous silica gel-H+ | 80 | 96 |
| mesoporeous silica gel-H+ | 100 | 92 |

### Example 5

500 ml (456.6 g, 2.87 mol) of DLL and 16 g of Amberlyst 15 were heated to 70° C. After 7 h reaction time the conversion was 92 % (control by gaschromatography). The mixture was destillated at 99.4 °C/147 mbar. There were thus obtained 370 g of ETTP (purity 97.32 %), corresponding to a yield of 82.4 %.

## Claims

1. Process for the preparation of compounds of the general formula I 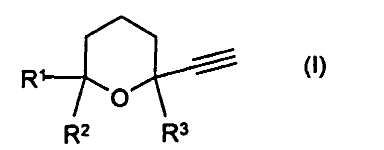 wherein R¹, R² and R³ are independently C₁- to C₇- alkyl,
which comprises exposing a compound of the general formula II 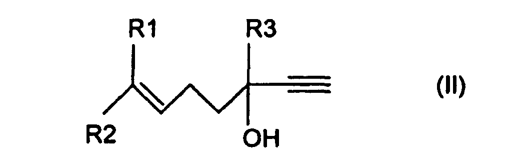 wherein R¹, R² and R³ are as above,
in the presence of a solid acid to temperatures of about 60°C to about 100°C or irradiation with microwaves.

2. A process as in claim 1, wherein a compound of formula II is irradiated with microwaves in the presence of a solid acid.

3. A process as in claim 1 or 2, wherein the solid acid is a zeolithe.

4. A process as in claim 3, wherein the zeolithe is Wessalith® Day P or Zeocat® Z6-05-02.

5. A process as in any one of claims 1 to 4, wherein the amount of solid acid used is about 0.5 to about 10, preferably about 1 to about 2 parts of weight per part of weight of compound of formula II.

6. A process as in any one of claims 1 to 5 wherein a microwave source of about 800 to about 1000 W is used.

7. A process as in any one of claims 1 to 6, wherein in the compound of formula II R¹, R² and R³ are methyl.

## Patentansprüche

1. Verfahren zur Herstellung von Verbindungen der allgemeinen Formel I 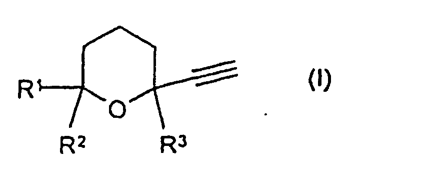 worin R¹, R² und R³ unabhängig C₁-C₇-Alkyl sind, das umfasst, dass eine Verbindung der allgemeinen Formel II 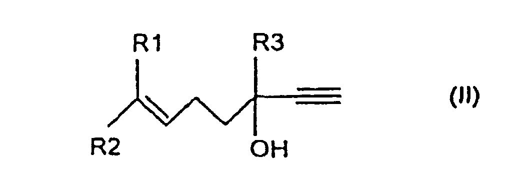 worin R¹, R² und R³ wie oben definiert sind, in Gegenwart einer festen Säure Temperaturen von etwa 60 bis etwa 100°C oder einer Bestrahlung mit Mikrowellen ausgesetzt wird.

2. Verfahren nach Anspruch 1, wobei eine Verbindung der Formel II mit Mikrowellen in Gegenwart einer festen Säure bestrahlt wird.

3. Verfahren nach Anspruch 1 oder 2, wobei die feste Säure ein Zeolith ist.

4. Verfahren nach Anspruch 3, wobei der Zeolith Wessalith® Day P oder Zeocat® Z6-05-02 ist.

5. Verfahren nach einem der Ansprüche 1 bis 4, wobei die Menge an fester Säure, die verwendet wird, etwa 0,5 bis etwa 10, bevorzugt etwa 1 bis etwa 2 Gewichtsteile pro Gewichtsteil der Verbindung der Formel II ist.

6. Verfahren nach einem der Ansprüche 1 bis 5, wobei eine Mikrowellenquelle mit etwa 800 bis etwa 1000 W verwendet wird.

7. Verfahren nach einem der Ansprüche 1 bis 6, wobei in der Verbindung der Formel II R¹, R² und R³ Methyl sind.

## Revendications

1. Procédé de préparation de composés de formule générale I : 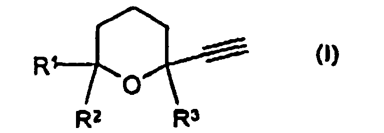 dans laquelle R¹, R² et R³ sont indépendamment des alkyles en C₁- à C₇-,
qui comprend l'exposition d'un composé de formule générale II 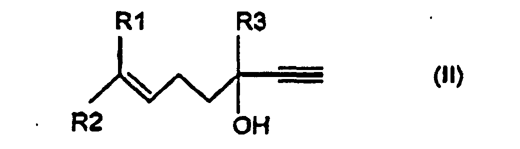 dans laquelle R¹, R² et R³ sont tels que ci-dessus,
en présence d'un acide solide à des températures d'environ 60°C à environ 100°C ou à l'irradiation par des micro-ondes.

2. Procédé selon la revendication 1, dans lequel un composé de formule II est irradié par des micro-ondes en présence d'un acide solide.

3. Procédé selon la revendication 1 ou 2, dans lequel l'acide solide est une zéolithe.

4. Procédé selon la revendication 3, dans lequel la zéolithe est la Wessalith® Day P ou la Zeocat® Z6-05-02.

5. Procédé selon l'une quelconque des revendications 1 à 4, dans lequel la quantité d'acide solide utilisée est d'environ 0,5 à environ 10, de préférence d'environ 1 à environ 2 parties en poids par partie en poids du composé de formule II.

6. Procédé selon l'une quelconque des revendications 1 à 5 dans lequel une source de micro-ondes d'environ 800 à environ 1000 W est utilisée.

7. Procédé selon l'une quelconque des revendications 1 à 6, dans lequel dans le composé de formule II, R¹, R² et R³ sont des méthyles.
